# EUROPEAN PATENT APPLICATION

(11) **EP 4 506 005 A1**
(43) Date of publication of application: **12.02.2025**
(21) Application number: 23190686.8
(22) Date of filing: 09.08.2023
(51) Int. Cl.: A61K 31/519, A61P 17/00, A61P 43/00

(54) **JAK-INHIBITOR FOR THE TREATMENT OF TEN, SJS AND SJS/TEN OVERLAP**

(71) Applicant: Max-Planck-Gesellschaft zur Förderung der Wissenschaften e.V., 80539 München (DE); Ludwig-Maximilians-Universität München, 80539 München (DE)
(72) Inventor: NORDMANN, Thierry, 82152 Martinsried (DE); MANN, Matthias, 82152 Martinsried (DE)
(74) Representative: Weickmann & Weickmann PartmbB

(57) **Abstract**

The present invention relates to a Janus kinase (JAK) inhibitor and to pharmaceutical composition comprising a Janus kinase (JAK) inhibitor, respectively, for use for the treatment of toxic epidermal necrolysis (TEN) and/or Stevens-Johnson-syndrome (SJS) and/or SJS/TEN overlap. The invention further relates to a method for treatment of toxic epidermal necrolysis (TEN) and/or Stevens-Johnson-syndrome (SJS) and/or SJS/TEN overlap comprising application of a Janus kinase (JAK) inhibitor to a patient.

## Description

The present invention relates to a Janus kinase (JAK) inhibitor and to a pharmaceutical composition comprising a Janus kinase (JAK) inhibitor, respectively, for use for the treatment of toxic epidermal necrolysis (TEN) and/or Stevens-Johnson-syndrome (SJS) and/or SJS/TEN overlap. The invention further relates to a method for treatment of toxic epidermal necrolysis (TEN) and/or Stevens-Johnson-syndrome (SJS) and/or SJS/TEN overlap comprising application of a Janus kinase (JAK) inhibitor to a patient.

Adverse drug reactions (ADRs) are frequent and a serious public health concern, comprising 7% of all hospital admissions [1, 2]. For reasons that remain unknown, the skin is one of the most common target organs of ADRs [3]. Cutaneous drug reactions occur often and contribute significantly to the morbidity of our society. Cutaneous ADRs range in severity from mild rashes, such as benign self-resolving maculo-papular rash (MPR), to potentially life-threatening drug reaction with eosinophilia and systemic symptoms (DRESS), Stevens-Johnson Syndrome (SJS) and Toxic Epidermal Necrolysis (TEN). Cutaneous ADRs affect up to 2% of hospitalized patients and are responsible for many chronic sequalae [3]. DRESS is a severe multiorgan hypersensitivity reaction triggered by culprit drugs and sustained by the reactivation of latent viruses, leading to aberrant T-cell and eosinophilic response. TEN and SJS are the most threatening and severe forms of cutaneous ADRs. SJS is considered a minor (<10%) variant of TEN (>30% body-surface skin detachment), and in between is considered overlap. In this document, all variants of TEN will be mentioned as TEN if not otherwise specified, for convenience of reading. Toxic epidermal necrolysis (TEN) is a fatal cutaneous adverse drug reaction and an emerging public health issue. Triggered by common medications, patients suffer from fulminant epidermal detachment and death or long-term sequalae. Although molecular mechanisms driving keratinocyte cytotoxicity have been reported, no effective therapy exists. TEN is a potentially lethal and rare mucocutaneous drug eruption, occurring at an estimated incidence of 2 to 7 cases per million population per year [4]. Fulminant epidermal detachment represents the hallmark of the disease. The overall mortality rate is high, ranging from 5% in SJS to 34% in TEN [5]. TEN results in substantial economic burden due to extensive costs for hospital care, chronic care and lost productivity of survivors [1, 2]. TEN is a T-cell mediated hypersensitivity reaction to a culprit drug in susceptible individuals. Specific drug-related HLA are well defined, emphasizing the role of genetic predisposition [6-9]. Drug-specific cytotoxic T cells expressing skin-homing receptors infiltrate the skin lesions in TEN and cytokines such as interferon-γ, IL-6, TNF-α, IL-18 and FasL are present in the involved epidermis of TEN-patients [10-15]. The exact mechanisms and therapeutic approaches are not yet fully understood. The mechanism of keratinocyte apoptosis and necroptosis leading to skin detachment in TEN is controversial and many mechanisms have been proposed, including FasL-Fas, granulysin, perforin/granzyme and monocyte-derived annexin-A1 [16-19]. Interestingly, the epidermal inflammatory infiltrate in TEN contains cells of the monocyte-macrophage lineage, which may act as an early driver of disease [20, 21]. Which cytokines are produced and how the innate immune response regulates inflammation and cell death caused by drugs in TEN in comparison to other cADR is poorly understood.

Consensus therapy of TEN is best supportive care. Current therapeutic strategies include high-dose intravenous immunoglobulins, TNF-α antagonists and cyclosporine [22, 23]. Reproducibly effective therapies do not exist. The precise molecular and intracellular signaling pathways in immune cells and the non-immune cell compartment are not known. To date, the standard of care for TEN is limited early cessation of the causative drug and best supportive care usually in an intensive care/burn unit. A strong evidence base for specific systemic therapy is lacking despite short-term in hospital mortality rates ranging from 15-20 percent.

It was therefore an objective of the present invention to resolve the molecular mechanism of cutaneous drug reactions and to provide active agents to effectively treat cutaneous adverse drug reactions, in particular toxic epidermal necrolysis (TEN) and/or Stevens-Johnson-syndrome (SJS) and/or SJS/TEN overlap.

It was found that Janus-kinase (JAK) inhibitors are effective in the treatment of toxic epidermal necrolysis (TEN) and/or Stevens-Johnson-syndrome (SJS) and or SJS/TEN overlap. Accordingly, the present invention relates to a Janus-kinase (JAK) inhibitor for use for the treatment of toxic epidermal necrolysis (TEN) and/or Stevens-Johnson-syndrome (SJS) and/or SJS/TEN overlap. Further, the invention relates to a pharmaceutical composition comprising a Janus-kinase (JAK) inhibitor for use for the treatment of toxic epidermal necrolysis (TEN) and/or Stevens-Johnson-syndrome (SJS) and/or SJS/TEN overlap. Further the invention relates to a method for treatment of toxic epidermal necrolysis (TEN) and/or Stevens-Johnson-syndrome (SJS) and/or SJS/TEN overlap comprising application of a Janus kinase (JAK) inhibitor to a patient.

TEN and SJS are severe forms of cutaneous ADRs. SJS is considered a variant of TEN having minor skin detachment (<10% body-surface skin detachment), while TEN is a more severe form having higher skin detachment (>30% body-surface skin detachment). The condition between SJS and TEN is designated SJS/TEN overlap herein having from 10 % body-surface skin detachment up to 30 % body-surface skin detachment. Herein, the term TEN is used to include all variants of TEN if not otherwise specified.

By studying the proteome of immune cells and keratinocytes in patients with various cutaneous adverse drug reactions, the inventors of the present application have found a dominant role of interferon signaling cascades and thus a therapeutic benefit of Janus kinase (JAK) inhibitors in patients with SJS/TEN. Surprisingly, the dominant role of interferon signaling cascades is specific for SJS/TEN and was not seen in seemingly similar other cutaneous adverse drug reactions such as DRESS or MPR.

The JAK-STAT pathway was identified as a critical component in the pathogenesis of TEN. This finding was in particular achieved by utilizing mass spectrometry-based techniques. Furthermore, JAK inhibitors were found and proven to dampen cytotoxic reactions and epidermal necrolysis occurring in in vitro and/or in vivo models of TEN. As a result, JAK inhibitors significantly alleviate disease manifestations during the acute phase of SJS-TEN. In cell culture experiments, the use of JAK inhibitors significantly reduced keratinocyte-directed cytotoxicity of activated peripheral blood cells from TEN patients. In addition, further validation in a mouse model was performed.

Unbiased spatial proteomics data on keratinocytes and immune cells from lesional skin biopsies obtained within the present invention, identified the JAK/STAT pathway as an actionable therapeutic target in toxic epidermal necrolysis. This pathway was successfully targeted with JAKi in a novel, live-cell imaging based in-vitro model using matched patient derived-keratinocytes and activated PBMCs to directly assess and perturbate keratinocyte-directed cytotoxicity. In combination with in-vivo inhibition in two mouse models of TEN this clearly demonstrated the biological relevance of the JAK/STAT pathway and the therapeutic efficacy of JAK inhibition in TEN.

In particular, it was shown that rapidly following the administration of a JAKi, macroscopic signs of disease severity of TEN can be markedly reduced. Furthermore, reduced disease severity was accompanied by a significant reduction in cutaneous pSTAT1 levels and average dermal thickness. Also, it was shown that JAKi also decreased keratinocyte cell death and immune cell infiltration. Importantly, epidermal recovery was accelerated by JAKi.

Thus, the present invention for the first time provides a conclusive functional evidence for the pathogenesis of SJS/TEN, based on proteomics, and confirmed both in-vitro by cell culture experiments and in-vivo by mouse experiments. Based thereon application of an JAK inhibitor as therapeutic agent for the treatment of SJS/TEN has been proven.

In particular, within the present invention, omic analysis, in particular proteomics of several cutaneous drug reactions, namely SJS/TEN, DRESS and MPR was performed concurrently. The data obtained clearly illustrate that the interferon signature is dominant in SJS/TEN both in comparison to healthy skin and also in comparison to other relevant cutaneous drug reactions, such as DRESS and MPR showing the importance of this pathway specifically for the disease SJS/TEN. As a result, the present invention identifies Janus-kinase (JAK) inhibitors as a treatment of toxic epidermal necrolysis (TEN) and/or Stevens-Johnson-syndrome (SJS) and/or SJS/TEN overlap.

### Janus Kinase (JAK) Inhibitors

Janus Kinases (JAK) are non-receptor tyrosine kinases. A Janus-kinase inhibitor (JAKi) as used herein includes any compound which inhibits and/or interferes with the JAK/STAT signaling pathway.

JAK inhibitors (JAKi) are already approved for various diseases, such as rheumatoid arthritis or atopic dermatitis, but have not yet been studied in the context of SJS/TEN.

Many cytokines, including interferon-α/β/γ, IL-6, IL-15 and IL-12 mediate their effects through the JAK pathway. Once activated, JAKs activate signal transducers and activators of transcription (STATs), leading to a plethora of transcriptional events. Given their central role in cytokine signaling, JAK inhibitors (JAKi) have been successfully applied in and approved for therapeutic use in various chronic inflammatory conditions, such as rheumatoid arthritis, inflammatory bowel disease, psoriasis and atopic dermatitis, amongst others [24]. In addition, JAKi have proven to be effective in acute inflammatory conditions such as graft versus host disease (GvHD) [25]. The usage of JAKi in this disease has been driven by discovery of interferon-γ mediated STAT1/3 activation at early stages of GvHD [26]. Interestingly, acute GvHD of severity grade IV and TEN can be challenging to differentiate, given the similar clinical presentation culminating in extensive skin blistering [27]. Yet, treatment regimens vary drastically. To date, JAKi have not been tested in TEN patients, possibly due to limited evidence on the role of interferon-γ and other cytokines that signal through the JAK/STAT pathway.

According to the invention it has now been found that Janus kinase (JAK) inhibitors can be used in the treatment of toxic epidermal necrolysis (TEN) and/or Stevens-Johnson-syndrome (SJS) and/or SJS/TEN overlap. According to the invention any JAKi can be used. Preferably a JAKi is used which is inhibiting JAK1, JAK2, JAK3 and/or TYK2. The Janus kinase (JAK) inhibitor is preferably selected from tofacitinib, baricitinib, ruxolitinib, upadacitinib, abrocitinib, cerdulatinib, deucravacitinib, fedratinib, lestaurtinib, momelotinib, pacritinib, peficitinib, delgocitinib, filgotinib, oclacitinib, ritlecitinib, brepocitinib, decernotinib, itacitinib, gandotinib or gusacitinib.

Preferably FDA-approved JAK inhibitors are employed, such as:
1. Tofacitinib (Xeljanz, from Pfizer), which can also be applied topically. Tofacitinib is targeted against JAK1-3. So far, the primary indication thereof is Rheumatoid arthritis.
2. Ruxolitinib (Jakafi, Jakavi from Incyte (U.S.) or Novartis (outside the U.S.) respectively), which can also be applied topically. Ruxolitinib is targeted against JAK1 and JAK2. So far, the primary indications thereof are Myelofibrosis and acute GvHD.
3. Baricitinib (Olumiant, from Eli Lilly). Baricitinib is targeted against JAK1-3. So far, the primary indication thereof is Rheumatoid arthritis.
4. Oclacitinib (Apoquel, from Zoetis). Oclacitinib is targeted against JAK1. So far, the primary indication is allergic dermatitis in dogs.
5. Fedratinib (Inrebic, from Celgene/Bristol Myers Squibb). Fedratinib is targeted against JAK2 and FLT3. So far, the primary indication is Myelofibrosis.
6. Upadacitinib (Rinvoq, from AbbVie). Upadacitinib is targeted against JAK1. So far, the primary indication is Rheumatoid arthritis.
7. Abrocitinib (from Pfizer). Abrocitinib is targeted against JAK1 and JAK2. So far, the primary indication is atopic dermatitis.
8. Delgocitinib (Corectim, from LEO Pharma and Japan Tobacco). Delgocitinib can be applied topically. Delgocitinib is targeted against pan-JAK / TYK2. So far, the primary indication is atopic dermatitis.
9. Filgotinib (Jyseleca, from Gilead Sciences and Galapagos NV). Filgotinib is targeted against JAK1. So far, the primary indication is rheumatoid arthritis.
10. Pacritinib (Vonjo, from CTI BioPharma). Pacritinib is targeted against JAK2 and FLT3. So far, the primary indication is Myelofibrosis.
11. Peficitinib (Smyraf, from Astellas Pharma). Peficitinib is a Pan-JAK inhibitor (JAK1, JAK2, JAK3, TYK2). So far, the primary indication is rheumatoid arthritis.
12. Ritlecitinib (Litulfo, from Pfizer). Ritlecitinib is a dual JAK3/TEC inhibitor (JAK3, TEC). So far, the primary indication is Alopezia Areata
13. Deucravacitinib (Sotyktu, from Bristol Myers Squib). Deucravacitinib is targeted against TYK2. So far, the primary indication is Psoriasis.

Further suitable JAK inhibitors include:
14. Brepocitinib (from Priovant Therapeutics), which can also be applied topically. Brepocitinib is targeted against JAK1 and TYK2. So far, the primary indication is Dermatomyositis.
15. Momelotinib (from GSK). Momelotinib is targeted against JAK1 and JAK2. So far, the primary indication is Myelofibrosis.
16. Cerdulatinib (from Portola Pharmaceuticals). Cerdulatinib is a pan-JAK/TYK2/SYK inhibitor. So far, the primary indication is peripheral T-cell lymphoma.
17. Decernotinib (VX-509, from Vertex Pharmaceuticals). Decernotinib is targeted against JAK3. So far, the primary indication is Rheumatoid arthritis.
18. Itacitinib (INCB039110, from Incyte). Itacitinib is targeted against JAK1. So far, the primary indication is graft-versus-host disease (GVHD).
19. Gandotinib (from Eli-Lily). Gandotinib is targeted against JAK2. So far, the primary indication is Myeloproliverate neoplasms.
20. Gusacitinib (ASN002, from Asana BioSciences). Gusacitinib is a pan-JAK/TYK2/SYK inhibitor. So far, the primary indication is Chronic eczema.

The Janus kinase (JAK) inhibitor of the invention is preferably inhibiting JAK1, JAK2, JAK3 and/or TYK2. Most preferred the Janus kinase (JAK) inhibitor is tofacitinib or Baricitinib, in particular Tofacitinib. Tofacitinib is a pan-JAKi, with inhibitory effects on all JAK-mediated signaling cascades. Tofacitinib exhibits high potency and long-lasting suppression of downstream inflammatory pathways. Furthermore, tofacitinib has a favorable pharmacokinetic profile, with peak plasma concentrations achieved within 30 to 60 minutes after administration.

### Proteomics/mass spectrometry

In recent years, powerful omics technologies have expanded into spatial context and single cell spatial proteomics was successfully applied in the present invention to uncover novel therapeutic targets in TEN.

Spatial omics holds the promise to address unmet clinical needs. Although a broad variety of technologies exists, translation into direct clinical benefit has remained largely aspirational. Limited by existing technologies and the rarity of severe cutaneous adverse reactions , it has not been possible to explore different skin-residing cellular subsets and multiple cutaneous drug reactions concurrently. Recently Deep Visual Proteomics (DVP) was developed as a novel spatial single-cell proteomics technology, leveraging high content imaging coupled with AI guided cell segmentation and classification, followed by laser micro dissection of individual target cells for ultra-sensitive proteomics. By using FFPE-archived biopsies, DVP enables high-yield, in-depth analysis of cells of interest, using accessible tissue specimens, even in rare disorders. In the present invention mechanisms of cutaneous drug reactions were studied by DVP, investigating the predominant molecular features in severe types and functional validation of selective small molecule inhibition.

Severe cutaneous adverse drug reactions (CADR) and in particular toxic epidermal necrolysis have elusive pathogenesis and cause substantial fatality. Through deep spatial proteomic profiling (DVP) of keratinocytes and immune cells from FFPE tissue sections, 5,000 proteins were quantified in keratinocytes and immune cells from 21 validated skin biopsies of three CADRs, namely MPR, DRESS and TEN. This in-depth proteomic analysis across all major types of cutaneous drug reactions with cell-type resolution provided unique insight into the molecular mechanism of each CADR and identified targets for therapeutic interventions of these disorders.

For instance, the striking upregulation of WARS1 in keratinocytes and immune cells of TEN, is of special interest in light of the increasingly appreciated role of the innate immune system in this disease. Both immune cells and keratinocytes carry functional TLR receptors, including TLR2 and 4. Thus, the results obtained link the non-canonical role of WARS1 as an endogenous TLR2/4 ligand to the immune pathogenesis of TEN. Given its ability to trigger cytokine and chemokine production, secreted WARS1 might directly contribute to the damage occurring in TEN by initiating and exacerbating the immune response.

In skin-infiltrating immune cells of DRESS, the most dominant s finding was the appearance of the histone methyltransferase EZH2, which was further confirmed by histology. EZH2 plays a key role in epigenetic gene regulation by modifying histone H3 at lysine 27 (H3K27me3). Functional consequences are thought to be proliferation of cancer cells, however, more recent studies have suggested a more nuanced, quantitative effect on defining cellular states. EZH2 is the third most common site of mutation (29%) in patients with idiopathic hypereosinophilic syndrome. Intriguingly, massive systemic eosinophilia is a hallmark of DRESS and the primary culprit of organ damage, providing a potential connection to EZH2. In addition, E2F pathway enrichment detected in the proteomic dataset correlates with the overexpression of EZH2, which functions as a downstream effector in this signaling cascade. Together, these findings strongly suggest that EZH2 and, by extension, epigenetic modifications, plays a significant part in the pathogenesis of DRESS. As EZH2 has can be inhibited by the FDA-approved drug Tazemetostat, elucidating the precise role of EZH2 in DRESS could open new therapeutic avenues for this severe, multiorgan inflammatory CADR.

The major finding of the present invention is the drastic upregulation of the JAK/STAT pathway in TEN on both proteome and transcriptome levels, which is not the case for the less severe drug reactions MPR or DRESS. The results pinpoint this signaling pathway as the main driver of cutaneous inflammation, keratinocyte cytotoxicity and epidermal detachment. Many components of the JAK/STAT signaling cascade are upregulated in both immune cells and keratinocytes of this disease, highlighting a potential synergistic interplay and an unfavorable feedback loop that could explain the extensive tissue damage. The cell type resolved findings of the present invention indicate a more active role of keratinocytes than previously anticipated in driving the skin directed cytotoxic immune response.

Applying Deep Visual Proteomics (DVP) to formalin fixed paraffin embedded archived skin-tissue biopsies of three types of cutaneous drug reactions, namely DRESS, MPR ant TEN, with varying severity quantified over 5,000 proteins in keratinocytes and skin-infiltrating immune cells. Most strikingly, this revealed a robust enrichment of Type-I- and - II interferon signature in the immune cell and keratinocyte compartment of TEN patients, along with a drastic activation of pSTAT1.

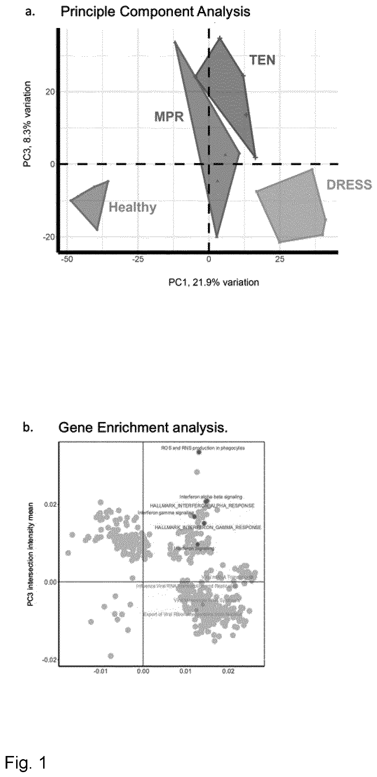

Using a novel mass-spectrometry based technology [28], the proteome of immune cells and keratinocytes in FFPE skin biopsies of 21 patients suffering from TEN, DRESS and MPR was analyzed in comparison to healthy skin. Using this approach, dimensionality reduction revealed a clear separation between all measured samples in the first dimension (Fig. 1a). To analyze the drivers of separation, a gene set enrichment analysis was performed with the ranked proteins of principle component 1 and 3. Terms for viral replication, synthesis and translation appeared as a prominent enrichment feature of DRESS patients, a disease in which herpes simplex virus reactivation acts as an underlying pathogenic mechanism and therefore confirms the validity of our workflow (Fig. 1a). In comparison, multiple terms showing a dominant interferon response and phagocyte involvement were present in patients suffering from TEN (Fig. 1b).

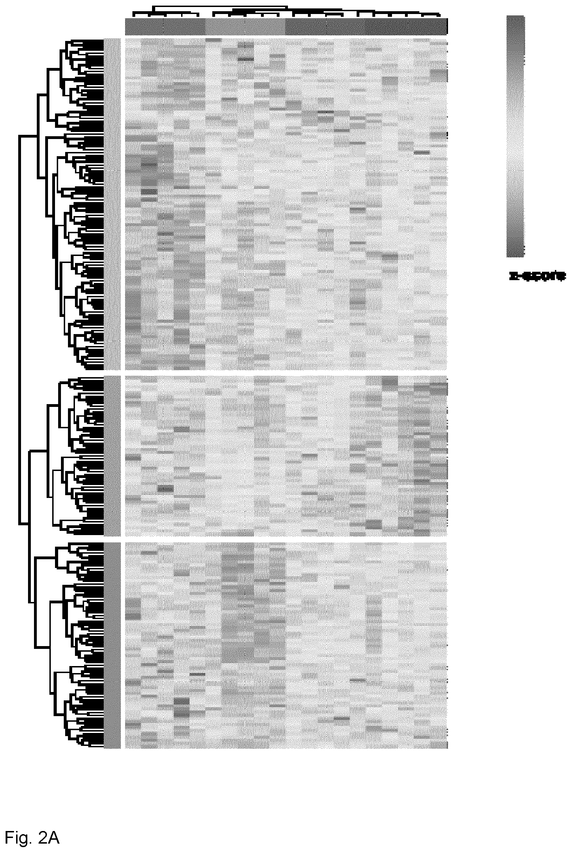

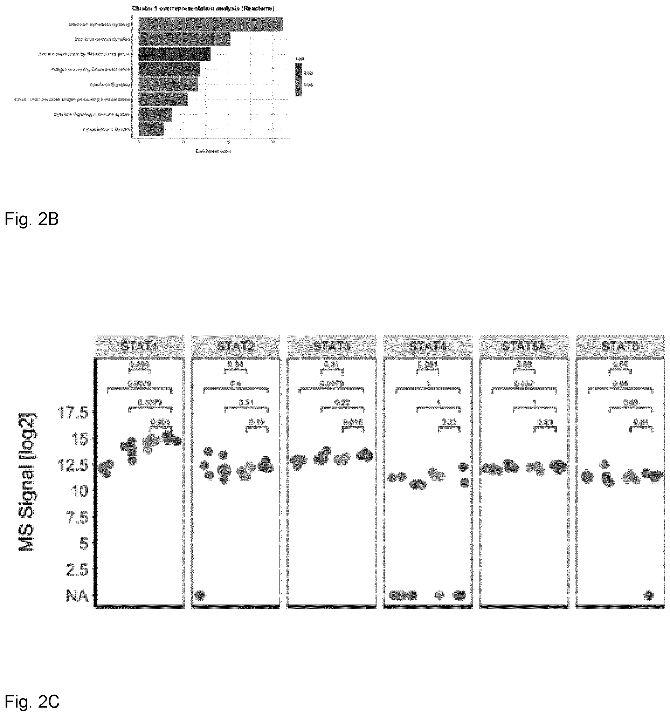

Further strengthening the concept of interferon-signaling as an essential mechanism in the pathogenesis of toxic epidermal necrolysis, is derived from analysis of covariance (ANOVA). Unsupervised hierarchical clustering of ANOVA significant proteins resulted in a clear and complete separation of disease phenotypes (Fig. 2A). Of specific interest was cluster 1, where protein levels were highest in patients with TEN. Gene set enrichment analysis for proteins of cluster 1 displayed interferon signaling pathways as a dominant feature (Fig. 2B). Using the power of MS-based proteomics, the different STAT proteins were further stratified within the immune cell compartment (Fig 2C). In immune cells of toxic epidermal necrolysis, STAT1 was significantly higher in comparison to all other conditions, while STAT1, STAT3 and STATS were significantly elevated in toxic epidermal necrolysis compared to healthy controls (Fig. 2C). STAT1 immunofluorescence staining confirmed these findings.

The deep visual proteomics data uncover the JAK-STAT and Type-I- and -II interferon signaling pathways as key pathogenic drivers of TEN and demonstrate the potential of targeted inhibition of JAK as a curative therapy. Thus, the JAK/STAT pathway was identified as actionable therapeutic target in toxic epidermal necrolysis by single cell spatial proteomics.

### In vitro assay/keratinocytes

The JAK/STAT pathway as an actionable therapeutic target in toxic epidermal necrolysis was successfully targeted with JAKi in a novel, live-cell imaging based in-vitro model using matched patient derived-keratinocytes and activated PBMCs to directly assess and perturbate keratinocyte-directed cytotoxicity.

The ability of JAK inhibition to suppress the cytotoxic reactions in-vitro was tested. Targeted inhibition with pan-JAK inhibitor (JAKi) Tofacitinib reduced keratinocyte-directed cytotoxicity in a novel, autologous live-cell imaging assay, using patient-derived keratinocytes and PBMCs.

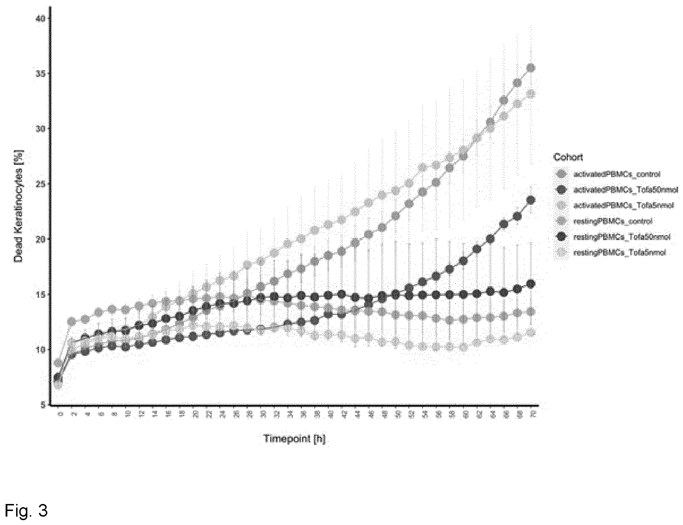

Upon co-culture of primary keratinocytes and peripheral PBMCs of a TEN survivor (culprit drug: Carbamazepine), Tofacitinib was able to dampen the keratinocyte mediated cytotoxicity of CD3/CD28 activated cells (Fig. 3). Importantly, non-activated (i.e. resting) PBMCs did induce cytotoxicity and there were no indications of increased cell death in the presence of Tofacitinib in combination with resting PBMCs.

### Mouse models of TEN

Further the effects of JAK inhibitors, such as Tofacitinib and other JAK inhibitors (including Baricitinib) were assessed in-vivo in a mouse model of TEN [29]. Oral administration of pan-JAKi Tofacitinib or Baricitinib ameliorated clinical and histological disease severity in a validated and published mouse model of TEN [29].

### Dosage range

Preferably the amount of JAKi inhibitor, in particular tofacitinib, comprised in the pharmaceutical composition of the present invention is from at least 0.1 mg, in particular of from at least 0.2 mg, preferably of from at least 1 mg, more preferably of from 2mg, even more preferably of from at least 5 mg up to 500 mg, in particular up to 50 mg, preferably up to 20 mg and more preferably up to 10 mg.

The Janus kinase (JAK) inhibitor is preferably administered in a daily amount of from 0,1 mg up to 500 mg, in particular up to 50 mg, preferably in a daily amount of from 1 mg up to 20 mg and more preferably in a daily amount of from 5 mg up to 20 mg. The total daily amount of JAKi is preferably administered once, twice or up to 4 times daily. Preferably the total daily amount of JAKi is administer in two daily doses. Most preferred the dosage is 10mg twice daily.

The JAKi is preferably administered in an acute phase SJS/TEN, in particular for one to ten, preferably for five days.

In a further preferred doses regime from the 6^{th} day on, preferably up to the 10^{th} day, a reduced daily amount of from 0.05 mg up to 50 mg, preferably from 1 mg up to 10 mg is administered.

Most preferred dosage is 10 mg orally twice for 5 days and then 5 mg orally twice for the next five days.

### Pharmaceutical compositions

According to the present invention, the Janus-kinase (JAK) inhibitor for use for the treatment of toxic epidermal necrolysis (TEN) and/or Stevens-Johnson-syndrome (SJS) and/or SJS/TEN overlap is in particular provided in form of a pharmaceutical composition. Such a pharmaceutical composition preferably comprises at least one Janus-kinase (JAK) inhibitor optionally together with a pharmaceutically acceptable carrier, vehicle, excipient and/or diluent.

"Pharmaceutical composition" refers to a preparation in a form that allows the biological activity of the active ingredient(s) to be effective, and which contains no additional components which are toxic to the patients to which the formulation is administered. As used herein, the term "pharmaceutically acceptable" means that the materials are suitable for administration to or upon a mammal, in particular a human, without production of undesirable physiological effects. "Pharmaceutically acceptable carrier" in particular refers to an ingredient in a pharmaceutical composition, other than an active ingredient, which is nontoxic to the subject to whom it is administered. A pharmaceutically acceptable carrier includes, but is not limited to, a buffer, excipient, stabilizer, or preservative.

The JAKi and the pharmaceutical composition comprising a JAKi, respectively, are preferably administered orally or topically, in particular orally.

For oral application the pharmaceutical composition can be formulated into powders, granules, tablets, capsules, suspensions, emulsions, syrups or any other oral dosage form. It can be prepared using a diluent or excipient such as generally used fillers, extenders, binders, wetting agents, disintegrating agents, surface active agents. A solid preparation for oral administration may be a tablet, pill, powder, granule, or capsule. The solid preparation may further comprise an excipient. Excipients may be, for example, starch, calcium carbonate, sucrose, lactose, or gelatine. In addition, the solid preparation may further comprise a lubricant, such as magnesium stearate, or talc.

For preparing solid compositions such as tablets, the JAKi can be mixed with a pharmaceutically acceptable carrier, e.g. conventional tableting ingredients such as corn starch, lactose, sucrose, sorbitol, talc, stearic acid, magnesium stearate, dicalcium phosphate or gums, and other pharmaceutical diluents, e.g. water, to form a solid pre-formulation composition containing a homogeneous mixture. In such a homogeneous pre-formulation compositions the JAKi is dispersed evenly throughout the composition so that the composition may be easily subdivided into equally effective unit dosage forms such as tablets, pills and capsules. This solid pre-formulation composition is then subdivided into unit dosage forms containing from 0.1 mg to about 500 mg of the active ingredient. Typical unit dosage forms contain from 1 mg to 100 mg, in particular from 2 mg, preferably from 5 mg and up 100 mg, preferably up to 50 mg und more preferably up to 20 mg of JAKi.

Liquid preparations for oral administration may be suspensions, solutions, emulsions, or syrups. The liquid formulation may comprise water, or liquid paraffin. The liquid formulation may, for example, include excipients, such as wetting agents, sweeteners, aromatics or preservatives. The liquid forms in which the composition of the present invention may be incorporated for administration orally include aqueous solutions, suitably flavored syrups, aqueous or oil suspensions, and flavored emulsions with edible oils such as cottonseed oil, sesame oil, coconut oil or peanut oil, as 15 well as elixirs and similar pharmaceutical vehicles. Suitable dispersing or suspending agents for aqueous suspensions include synthetic and natural gums such as tragacanth, acacia, alginate, dextran, sodium carboxymethylcellulose, Methylcellulose, polyvinylpyrrolidone or gelatin.

### Combination therapy / Inhibitors with multiple targets.

The present invention further relates to a pharmaceutical composition for use for the treatment of toxic epidermal necrolysis (TEN) and/or Stevens-Johnson-syndrome (SJS) and/or SJS/TEN overlap comprising a JAK inhibitor, wherein the pharmaceutical composition causes simultaneous inhibition of multiple pathways, including the JAK/STAT pathway.

In a preferred embodiment of the invention a compound is employed being a Jak inhibitor, which also causes inhibition of at least one further pathway. Thereby additional benefit for the patients can be achieved. Preferred examples of such JAKi inhibitiors also causing inhibition of at least one further pathway are Ritlecitinib, Cerdulatinib or Gusacitinib.

Ritlecitinib (Litulfo, from Pfizer). Ritlecitinib is a dual JAK3 /TEC (tyrosine kinase expressed in hepatocellular carcinoma) kinase family inhibitor. So far, the primary indication is Alopecia Areata. (TEC) family of protein kinases comprise five members: Bruton's tyrosine kinase (BTK), bone marrow tyrosine kinase on chromosome X (BMX), interleukin 2-inducible T cell kinase (ITK), resting lymphocyte kinase (RLK), and TEC.

Cerdulatinib (from Portola Pharmaceuticals). Cerdulatinib is a dual pan-JAK-TYK2 / SYK (spleen tyrosin kinase) inhibitor. So far, the primary indication is peripheral T-cell lymphoma. SYC is a tyrosine kinase and mediates multiple signaling pathways, including inflammatory ones, such as PI3K amongst other.

Gusacitinib (ASN002, from Asana BioSciences). Gusacitinib is a dual pan-JAK-TYK2/SYK inhibitor. So far, the primary indication is Chronic eczema.

By now, BTK, SYK, FLT3, HAD, Src, and Aurora kinases have been developed as dual inhibitors (in combination with JAK inhibitors), leveraging the synergistic effects of dual JAK inhibitors over single inhibition.

Dual JAK and TYK2 inhibition is regarded as direct inhibition of the JAK/STAT pathway, as opposed to other dual inhibitors that target the JAK/STAT and an additional pathway outside of this.

The present invention further relates to a pharmaceutical composition for use for the treatment of toxic epidermal necrolysis (TEN) and/or Stevens-Johnson-syndrome (SJS) and/or SJS/TEN overlap comprising a JAK inhibitor, which comprises at least one additional pharmaceutically active agent. Thus, the invention also includes combination therapy combining an JAK inhibitor with at least one further pharmaceutically active agent.

In particular the JAKi can be combined with ivlGs (intravenous immunoglobulins), with TNFα-inhibitors, with corticosteroids and/or with cyclosporin.

Herein also described is a novel co-culture, live-cell imaging assay comprising matched keratinocytes and immune cells which are in particular derived from the same patient. This imaging assay is in particular suitable for cutaneous, immune-mediated processes in general and in particular for in detail molecular analysis of cutaneous drug reactions.

The present application in particular discloses an imaging assay for analysis of immune-mediated processes and in particular for analysis of cutaneous drug reactions comprising
(i) generating hair follicle-derived keratinocytes;
(ii) isolating peripheral blood mononuclear cells (PBMCs) or subsets of PBMCs;
(iii) activating the isolated PBMCs or subsets of PBMCs;
(iv) labelling the keratinocytes;
(v) co-culturing labelled keratinocytes
   a) with activated PBMCs or subsets of PBMCs and
   b) alternatively with resting PBMCs or subsets of PBMCs;
(vi) identifying killed keratinocytes.

In step (i) the hair follicle-derived keratinocytes from a patient are in particular cultivated. In step (iii) activating the isolated PBMCs or subsets of PBMCs can be performed with CD3/28. Preferably, activation is performed with the drugs causing the adverse drug reaction. In step (iv) the keratinocytes are preferably fluorescently labelled. Then, keratinocytes are co-cultured with activated PBMCs or subsets of PBMCs and in the alternative with resting PBMCs or subsets of PBMCs. Cytotoxic action of activated PBMCs or subsets of PBMCs toward keratinocytes can be visualized using live-cell imaging. In a positive control, keratinocytes are efficiently killed in the presence of activated PBMCs or subsets of PBMCs while they should not be affected by resting PBMCs or subsets of PBMCs. For testing a drug for efficiency in this assay the drug or active agent to be tested is added to step (v). Thereby, dose - dependent inhibition of keratinocyte cytotoxity of the respectice active agent can be evaluated.

### References

1. Lazarou, J., B.H. Pomeranz, and P.N. Corey, Incidence of adverse drug reactions in hospitalized patients: a meta-analysis of prospective studies. JAMA, 1998. 279(15): p. 1200-5.
2. Hsu, D.Y., et al., Morbidity and Mortality of Stevens-Johnson Syndrome and Toxic Epidermal Necrolysis in United States Adults. J Invest Dermatol, 2016. 136(7): p. 1387-1397.
3. Valeyrie-Allanore, L., B. Sassolas, and J.C. Roujeau, Drug-induced skin, nail and hair disorders. Drug Saf, 2007. 30(11): p. 1011-30.
4. Chung, W.H., C.W. Wang, and R.L. Dao, Severe cutaneous adverse drug reactions. J Dermatol, 2016. 43(7): p. 758-66.
5. Sekula, P., et al., Comprehensive survival analysis of a cohort of patients with Stevens-Johnson syndrome and toxic epidermal necrolysis. J Invest Dermatol, 2013. 133(5): p. 1197-204.
6. Hung, S.I., et al., HLA-B*5801 allele as a genetic marker for severe cutaneous adverse reactions caused by allopurinol. Proc Natl Acad Sci USA, 2005. 102(11): p. 4134-9.
7. Chung, W.H., S.I. Hung, and Y.T. Chen, Human leukocyte antigens and drug hypersensitivity. Curr Opin Allergy Clin Immunol, 2007. 7(4): p. 317-23.
8. Chung, W.H., et al., Medical genetics: a marker for Stevens-Johnson syndrome. Nature, 2004. 428(6982): p. 486.
9. Ko, T.M., et al., Shared and restricted T-cell receptor use is crucial for carbamazepine-induced Stevens-Johnson syndrome. J Allergy Clin Immunol, 2011. 128(6): p. 1266-1276 e11.
10. Nassif, A., et al., Toxic epidermal necrolysis: effector cells are drug-specific cytotoxic T cells. J Allergy Clin Immunol, 2004. 114(5): p. 1209-15.
11. Le Cleach, L., et al., Blister fluid T lymphocytes during toxic epidermal necrolysis are functional cytotoxic cells which express human natural killer (NK) inhibitory receptors. Clin Exp Immunol, 2000. 119(1): p. 225-30.
12. Friedmann, P.S., et al., Investigation of mechanisms in toxic epidermal necrolysis induced by carbamazepine. Arch Dermatol, 1994. 130(5): p. 598-604.
13. Villada, G., et al., Immunopathology of toxic epidermal necrolysis. Keratinocytes, HLA-DR expression, Langerhans cells, and mononuclear cells: an immunopathologic study of five cases. Arch Dermatol, 1992. 128(1): p. 50-3.
14. Heng, M.C. and S.G. Allen, Efficacy of cyclophosphamide in toxic epidermal necrolysis. Clinical and pathophysiologic aspects. J Am Acad Dermatol, 1991. 25(5 Pt 1): p. 778-86.
15. Correia, O., et al., Cutaneous T-cell recruitment in toxic epidermal necrolysis. Further evidence of CD8+ lymphocyte involvement. Arch Dermatol, 1993. 129(4): p. 466-8.
16. Paul, C., et al., Apoptosis as a mechanism of keratinocyte death in toxic epidermal necrolysis. Br J Dermatol, 1996. 134(4): p. 710-4.
17. Saito, N., et al., An annexin A1-FPR1 interaction contributes to necroptosis of keratinocytes in severe cutaneous adverse drug reactions. Sci Transl Med, 2014. 6(245): p. 245ra95.
18. Viard-Leveugle, I., et al., TNF-alpha and IFN-gamma are potential inducers of Fas-mediated keratinocyte apoptosis through activation of inducible nitric oxide synthase in toxic epidermal necrolysis. J Invest Dermatol, 2013. 133(2): p. 489-98.
19. Chung, W.H., et al., Granulysin is a key mediator for disseminated keratinocyte death in Stevens-Johnson syndrome and toxic epidermal necrolysis. Nat Med, 2008. 14(12): p. 1343-50.
20. Roujeau, J.C., et al., Involvement of macrophages in the pathology of toxic epidermal necrolysis. Br J Dermatol, 1985. 113(4): p. 425-30.
21. Paquet, P., et al., Macrophages and tumor necrosis factor alpha in toxic epidermal necrolysis. Arch Dermatol, 1994. 130(5): p. 605-8.
22. Viard, I., et al., Inhibition of toxic epidermal necrolysis by blockade of CD95 with human intravenous immunoglobulin. Science, 1998. 282(5388): p. 490-3.
23. Wang, C.W., et al., Randomized, controlled trial of TNF-alpha antagonist in CTL-mediated severe cutaneous adverse reactions. J Clin Invest, 2018. 128(3): p. 985-996.
24. Spinelli, F.R., et al., JAK inhibitors: Ten years after. Eur J Immunol, 2021. 51(7): p. 1615-1627.
25. Braun, L.M. and R. Zeiser, Kinase Inhibition as Treatment for Acute and Chronic Graft-Versus-Host Disease. Front Immunol, 2021. 12: p. 760199.
26. Ma, H.H., et al., Sequential activation of inflammatory signaling pathways during graft-versus-host disease (GVHD): early role for STAT1 and STAT3. Cell Immunol, 2011. 268(1): p. 37-46.
27. Jeanmonod, P., et al., Graft-versus-host disease or toxic epidermal necrolysis: diagnostic dilemma after liver transplantation. Transpl Infect Dis, 2012. 14(4): p. 422-6.
28. Mund, A., et al., Deep Visual Proteomics defines single-cell identity and heterogeneity. Nat Biotechnol, 2022. 40(8): p. 1231-1240.
29. Anderton, H., et al., Inhibitor of Apoptosis Proteins (lAPs) Limit RIPK1-Mediated Skin Inflammation. J Invest Dermatol, 2017. 137(11): p. 2371-2379.

The present invention is further illustrated by the appended figures.
Fig. 1. Principal component analysis of immune cell compartment
   A) Principal Component Analysis (PCA) of 21 samples corresponding to the CD45+ compartment of the respective skin sample based on their proteomic expression profiles (mean of duplicates). The first and third dimensions segregate the samples and account for 21.9% and 8.3% of the total variability, respectively. The color code corresponds to the four disease groups. B) Gene set enrichment analysis (Benjamini-Hochberg FDR < 0.05, GOBP terms) are shown exemplarily for co-expressed enrichment terms across principal component 1 and 3.
Fig. 2. Proteome of immune cell compartment
   A) Heatmap of normalized protein abundances of differentially expressed proteins (ANOVA, FDR < 0.05) upon unsupervised hierarchical clustering. Up- and downregulated proteins are represented in red and blue, respectively. B) Overrepresentation analysis (Benjamini-Hochberg FDR < 0.05, Reactome terms) are shown for cluster 1 of Fig. 2A. C) Log2 MS intensity values of identified STAT proteins. Numbers indicate p-value of unpaired t-test per protein. Color indicates cohort: grey = healthy, blue = MPR, orange = DRESS, purple = TEN.
Fig. 3. Tofacitinib ameliorates T-cell mediated keratinocyte cytotoxicity in-vitro
   Percentage of CelltoxTM Green positive (i.e. dead) keratinocytes (y-axis) over time (x-axis) upon coculture of 10⁴ primary keratinocytes with 10⁴ CD3/28 activated or non-activated (i.e. resting) PBMCs in the presence of either 50nmol or 5nmol Tofacitinib. Images were acquired every 2h (Incucyte^{®}). Dots represent mean of n = 6 per timepoint, error-bar represent s.e.m.

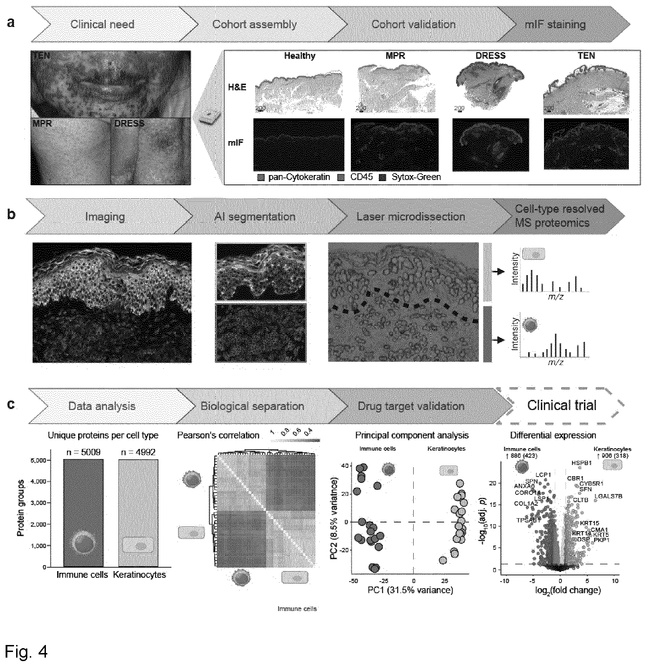

Fig. 4 | Deep Visual Proteomic workflow for in-depth proteomic data with cellular resolution in cutaneous adverse drug reactions. a. Cohort assembly and validation (N=21). Hematoxylin & Eosin (H&E) and multicolor immunofluorescence staining of a representative 3um FFPE tissue section from the indicated conditions (red: pan-cytokeratin+ keratinocytes, purple: CD45+ immune cells, blue: sytox-green+ nuclei). Maculopapular rash (MPR), drug rash with eosinophilia and systemic symptoms (DRESS), toxic epidermal necrolysis (TEN) and healthy individuals. N = 5-7 individuals per cohort, b. The Deep Visual Proteomics pipeline, including high-content imaging, machine learning-based cell segmentation and classification, followed by laser microdissection and ultra-sensitive mass spectrometry-based proteomics at cellular resolution. Representative area of excised keratinocyte (yellow arrow) and immune cell (red arrow) from FFPE tissue sections. c. Identification of unique protein groups, Pearson correlation, principal component analysis and differentially expressed proteins (DEP) quantified across all patients in the indicated cell type. Colored dots are significant (BH adjusted p < 0.05, FDR 0.05), dashed line represents log2FC of ≤ 1 or ≥ 1. N = 5 to 7 per condition, mean of biological duplicates per patient. [Biorender].

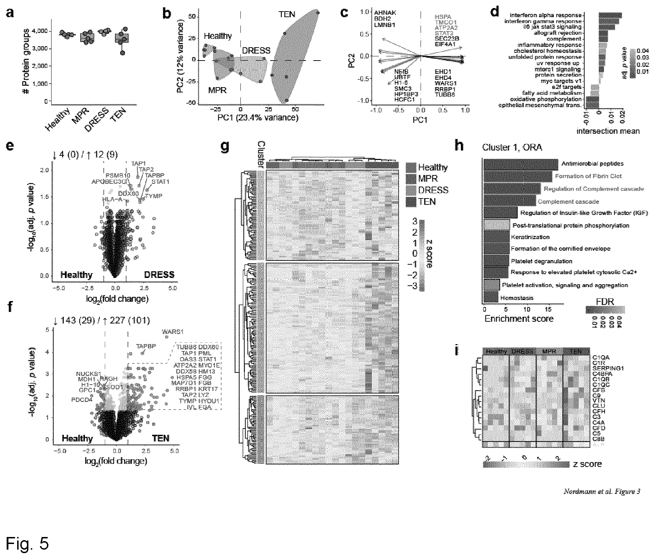

Fig. 5 | a. Number of proteins identified in keratinocytes the indicated conditions, b. Principal Component (PCA) analysis, c. main drivers of separation and d. PC1 gene set enrichment analysis. e-f. Differentially expressed proteins between the indicated condition and healthy. Colored dots are significant (BH adjusted p.value < 0.05, FDR 0.05), dashed vertical line represents log2FC of ≤ 1 or ≥ 1. g. Unsupervised hierarchical clustering with analysis of variance (ANOVA) significant proteins (adjusted p. value < 0.05, FDR < 0.05, post-hoc Tukey HSD). Color indicates normalized values (Z-score) h. Overrepresentation analysis of ANOVA cluster 1, ordered according to enrichment score. Color indicates degree of significance (FDR). i. Semisupervised heatmap of complement factors in the indicated conditions, and albumin (at the bottom) to exclude serum contamination, Color indicates normalized values (Z-score). N = 5 to 7 per condition, mean of biological duplicates per patient.

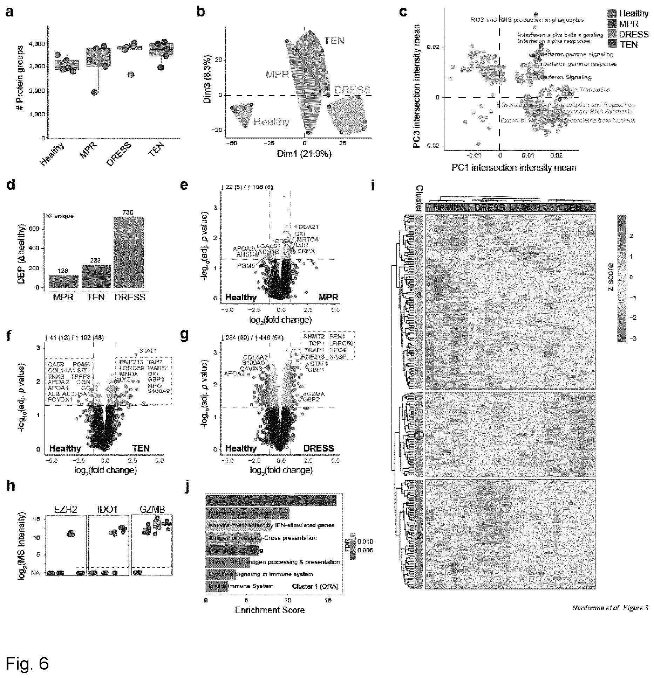

Fig. 6| a. Number of proteins identified in immune cells in the indicated conditions, b. Principal Component (PCA) analysis of the proteomic results. c. Intersection of PC1 and PC3 gene set enrichment analysis. Terms including "viral" or "interferon" are highlighted d-g. Differentially expressed proteins in the indicated conditions versus healthy and corresponding volcano plots. Shaded area within columns represents the proportion unique proteins. In volcano plots, color represents significance (BH adjusted p < 0.05, FDR 0.05), dashed vertical line represents log2FC of ≤ 1 or ≥ 1. h. Selected proteins of interest, with a distinct distribution across the cohorts i. Unsupervised hierarchical clustering of ANOVA significant proteins (adjusted p. value < 0.05, FDR < 0.05, post-hoc Tukey HSD). Color indicates normalized values (Z-score). j. Overrepresentation analysis of ANOVA cluster 1, ordered according to enrichment score. Color indicates degree of significance (FDR). N = 5 to 7 per condition, mean of biological duplicates per patient.

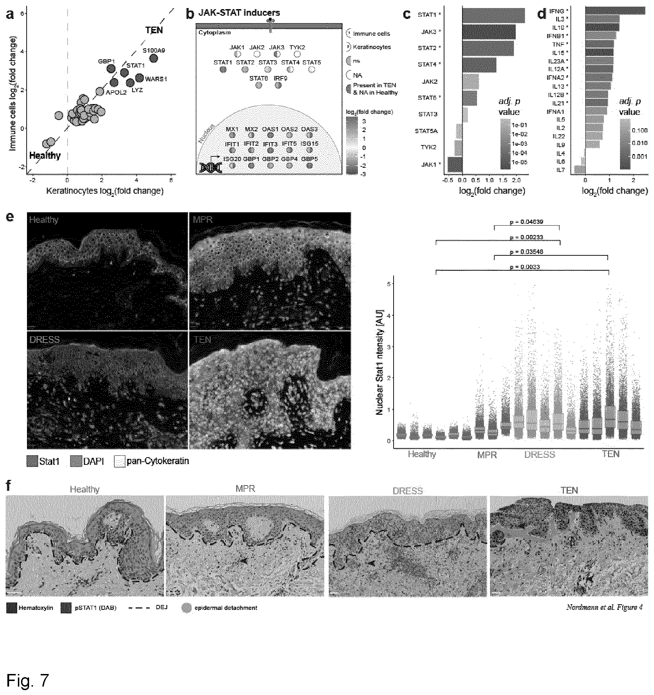

Fig. 7 | a. Significantly quantified keratinocyte (x-axis) and immune cell (y-axis) proteins and their corresponding log2 fold change (log2FC) compared to healthy. Purple color indicates proteins with log2FC > 2 in both cell types. Dashed vertical and horizontal lines indicate no fold change and the dashed diagonal line represents equal regulation. b. Schematic illustration of quantified JAK/STAT pathway proteins in both cell types. Color represents log2FC compared to healthy, with direct comparison between immune cells (left semi-circle) and keratinocytes (right) of the indicated protein. Empty (semi-)circles are proteins with missing values. All colors except for grey are significantly regulated (q < 0.05). c. mRNA expression levels in SJS/TEN compared to healthy for the indicated genes of the JAK/STAT pathway (validation cohort) d. mRNA expression change in TEN compared to healthy of genes signaling through the JAK/STAT pathway (validation cohort). Asterisks indicate significance (q < 0.05). e. Representative immunofluorescent staining of FFPE tissue sections against Stat1 (red) and pan-Cytokeratin (yellow). Quantification of nuclear Stat1 intensity per cell, normalized to its own nuclear staining intensity, for all samples of the corresponding conditions, f. Representative phospho-STAT1 immunohistochemistry staining. Dashed lines represent the dermo-epidermal junction (DEJ). Epidermal detachment is highlighted with red (in TEN).

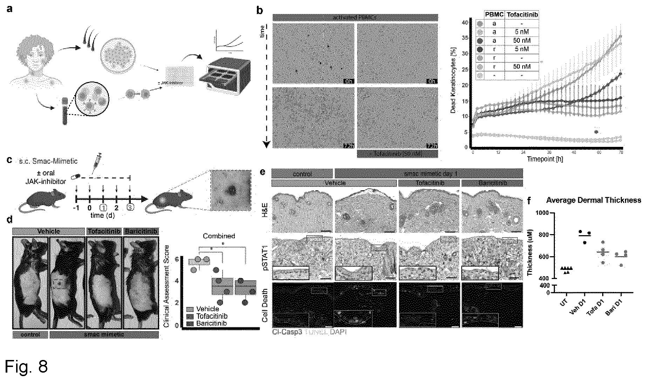

Fig. 8 | a. Live-cell imaging co-culture assay with keratinocytes and activated PBMCs from the same patient ± JAK inhibitor, to assess keratinocyte cell death b. Keratinocytes labeled with CellTracker Red (arrow) in co-culture with unlabeled PBMCs (arrowhead) at timepoint 0 and 72h, ± 50nM Tofacitinib. Kinetic data of the corresponding conditions quantified over time (a = activated; r = resting; n=6 replicates per condition) c. Experimental protocol to assess efficacy of oral JAK inhibitor Tofacitinib (30 mg/kg) or Baricitinib (10 mg/kg) in combination with s.c. smac mimetics, which cause a TEN phenotype (red arrow: epidermal detachment). d. Representative macroscopic image and clinical assessment score to evaluate the macroscopic effect of treatment of the corresponding cohort on day three. e. Representative H&E, pSTAT1 and cell death (red = cleaved caspase 3 / yellow = TUNEL) microscopy image of the corresponding cohort. Inserts magnify area of interest, f. Average dermal thickness of the indicated conditions (n = 4-6).

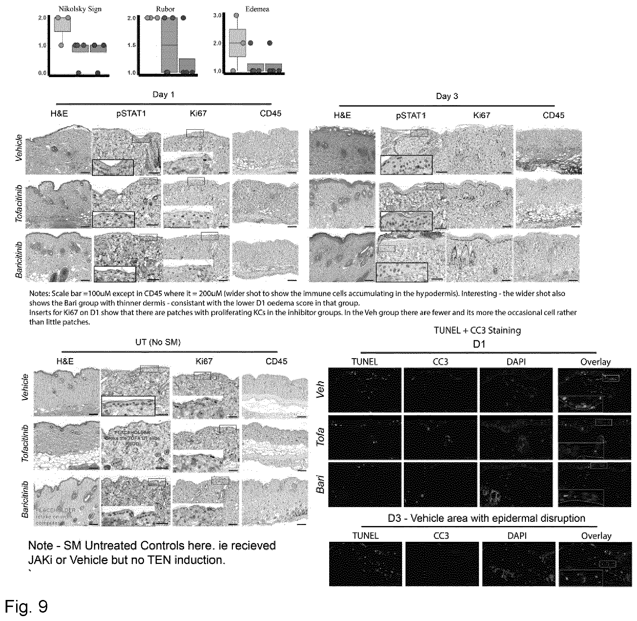

Fig. 9 | Further macroscopic and histologic assessment of the smac mimetic mouse model. a. Subscores of combined score (Fig. 8d) to evaluate the macroscopic effect of treatment in the smac mimetic mouse model described above. b. Histological assessment of H&E, pSTAT1, Ki67, CD45 and CC3/TUNEL staining in the indicated conditions within the smac mimetic mouse model. Inserts magnify area of interest, f. Average dermal thickness of the indicated conditions (n = 4-6).

### Example 1 Proteomics

### 1.1 Cohort assembly and spatial proteomics workflow

To study molecular mechanisms of cutaneous drug reactions, an age and gender matched, retrospective cohort of FFPE lesional skin biopsy samples from patient with mild- (maculo-papular rash-MPR) or severe-CADR (TEN, DRESS) and healthy control individuals (Fig. 4a, N = 21) was assembled.

Skin tissue biopsies were obtained during routine histopathological diagnostic procedures at the Departments of Dermatology and Allergology of the University Hospital Zurich (proteomic cohort) and Munich University of Ludwig Maximilian (validation cohort). In the proteomic cohort, TEN patients had a minimal affected skin area of 30%. In the validation cohorts, patients with SJS/TEN overlap were included (minimal affected skin >10%). All patients with DRESS had a RegiSCORE of 6-7 (definite diagnosis). In addition to the typical clinical features, the lymphocyte transformation test (LTT) was positive in all MPR patients. Histopathological diagnosis was re-validated in all cases by a board-certified dermatopathologist and dermatologist using a fresh H&E-stained tissue section. Baseline characteristics were statistically evaluated using Analysis of Variance (ANOVA) for numeric variables (age) and a Chi-squared test of independence for categorical variables (gender). All obtained disease biopsies were performed in the context of routine clinical workup for retrospective analysis. Ethical approval is present for all collected samples.

After clinical and histological re-validation of all cases, 3µm FFPE tissue sections were stained for CD45 to label immune cells and with pan-cytokeratin for keratinocytes (Fig. 4b). A machine learning (ML) algorithm ensured proper segmentation and prevented downstream analysis of overlapping cellular compartments. Thereafter, contours of immune cells and keratinocytes from each patient were laser micro-dissected and each separately pooled into multi-well plates for subsequent peptide extraction and ultra-high sensitivity MS-data acquisition (Fig. 4b). This DVP approach achieved a proteomic depth of 4992 unique proteins in immune cells and 5009 in keratinocytes across all patients (Fig. 4c).

The biological separation of keratinocytes from immune cells greatly exceeded interpatient heterogeneity (Fig. 4c). A third of all identified proteins were differentially expressed, including the expected upregulation of LGALS7B and KRT5 in keratinocytes and of ANXA6 and CORO1A in immune cells, confirming the cell-type resolution of the DVP data (Fig 4c).

### 1.2 Distinct proteome of keratinocytes in drug reactions

Having acquired the cell-type resolved proteomes of the cohort, comprehensive data analysis of keratinocytes involved in the selected CADRs was performed. A median of 3788 protein groups was quantified across all patients, covering a dynamic range of 4.2 orders of magnitude (Fig. 5a). Data completeness and quantitative accuracy were excellent (median coefficient of variation (CV) of 17.4 % per cohort, despite patient heterogeneity). Keratinocyte proteomes clustered according to the type of drug reaction, with Principal Component 1 separating TEN from other CADR and healthy (Fig. 5b). PC1 was enriched with proteins involved in calcium homeostasis and cellular stress response, including HSP5 (heat-shock protein family A member 5), TMCO1 (transmembrane and coiled coil domains 1) and ATP2A2 (ATPase sarcoplasmic/endoplasmic reticulum Ca2+ transporting 2; Fig. 5c). Interestingly, STAT3 (signal transducer and activator of transcription 3) was amongst the main drivers of PC1, congruent with a dominant interferon signature in gene set enrichment analysis of PC1 (GSEA; Fig. 5d). Pairwise comparison of diseased to healthy proteomes revealed an increasing number of differentially expressed proteins (DEP) following the severity of the CADR. MPR, the mildest CADR, had no significantly regulated proteins compared to healthy, while DRESS robustly upregulated multiple MHC class-I proteins in keratinocytes, suggesting that keratinocytes participate actively in culprit-drug associated antigen presentation (TAP1, TAP2, TAPBP, HLA-A; log2FC > 1, Fig. 5e).

In keratinocytes of TEN, the most severe CADR, there were 227 up - and 143 significantly down-regulated proteins, including antimicrobials such as Lysozyme and Clusterin (Fig. 5f). WARS1, one of the most strongly upregulated proteins -, has not previously been associated with TEN (log2FC 4.36, q 2.04 x 10-5). This tryptophanyl-tRNA Synthetase has a non-canonical role in activating the innate immune system as an endogenous TLR2/4 ligand, thereby inducing cytokine and chemokine production. Expression of superoxide-Dismutase (SOD) 1 was reduced by half, which is interesting because oxidative stress and reactive oxygen species (ROS) are known to upregulate keratinocyte Fas-ligand expression, a key cytolytic molecule involved in keratinocyte apoptosis and epidermal detachment in TEN. Unsupervised hierarchical clustering of ANOVA significant proteins between all three CADR types, showed a clear alignment for TEN and three distinct protein clusters, revealing a further degree of stratification (Fig. 5g). Overrepresentation analysis of cluster 1, which represents proteins with highest normalized intensity values in TEN, revealed multiple terms involved in the regulation of complement activation and blood clotting (Fig. 5h). Nearly all complement factors in our keratinocyte proteome dataset are uniquely upregulated in keratinocytes of TEN (Fig. 2i).

### 1.3 The proteome of immune cells in drug reactions

Cutaneous immune cell infiltration in TEN is thought to be sparse and scattered, in contrast to the dense and confined perivascular infiltrates in MPR and DRESS29. To understand underlying molecular differences in immune cells of the skin in CADR, the in-depth proteome of immune cells (median of 3418 per patient, median cv 19.8 % within cohorts, was assessed with high data completeness; (Fig. 6a). These proteomes again clustered according to disease phenotype but in contrast to keratinocytes, Principal Component 1 of immune cells already separated DRESS from other CADR and healthy (Fig. 6b). Gene set enrichment analysis of PC1 was prominently associated with E2F and MYC targets and proteins driving this include RNA processing and splicing factors such as DDX39A (DExD-Box Helicase 39A), EIF4A3 (Eukaryotic Translation Initiation Factor 4A3) and SRSF6 (Serine and Arginine Rich Splicing Factor 6), indicating a highly proliferative state. PC1 and PC3 together revealed unique features of DRESS and TEN: in DRESS, multiple viral pathways terms are enriched, in line with the postulated role of latent viral reactivation in its pathogenesis. In contrast, a dominant interferon signature was evident in TEN, together with STAT1 as a main driver. (Fig. 6c). In direct proteomic comparison to healthy, DRESS had the highest number of differentially expressed proteins (DEP, n = 730), followed by TEN (n = 233) and MPR (n = 128; Fig. 3d - g). It was focused on the DEPs unique to TEN, which included GCA (Grancalcin, log2FC 1.56, q 2.38 x 10-2) and PDIA3 (Protein Disulfide Isomerase Family A Member 3, log2FC 0.642, q 2.51 x 10-2), both not previously described in the context of TEN. Similar to its role in cancer, PDIA3 may modulate MHC-class I and subsequent CD8+-driven cytotoxicity in TEN.

A minority of proteins were specifically associated with defined CADR subsets. Of particular interest is histone methylase EZH2 (Enhancer of Zeste homolog), the catalytic member of PRC (Polycomb Repressive Complex) 2, detectable only in immune cells of DRESS (Fig. 6h). Immunofluorescence staining, as well as near-exclusive transcription in a data set of PBMC-derived lymphocyte cluster from a therapy resistant DRESS patient, confirmed our proteomic observation. In fact, EZH2 fold change over healthy was higher than the main targets reported in that study (STAT1, JAK3). EZH2's role in cancer has led to the development of an FDA-approved inhibitor, its contribution to inflammatory and allergic diseases is hardly investigated.

IDO (Indoleamine 2, 3-dioxygenase 1) was only detected in severe CADR and represents a further targetable protein (Fig. 6h). IDO1 might have a detrimental role or might dampen the cytotoxic reaction, as within the tumor microenvironment. Although Granzyme B has been described as a key mediator of cytotoxicity in TEN25, our proteomics data show that it is present at similar levels in non-cytotoxic DRESS (Fig. 6h).

Unsupervised hierarchical clustering of ANOVA significant proteins in immune cells resulted in three visually distinct protein clusters with perfect disease alignment (Fig. 6i). Overrepresentation analysis of Cluster 2, which mainly represents DRESS, confirmed the highly replicative features of infiltrating immune cells in this disease, illustrated by multiple terms of DNA replication. Its most prominent term (Activation of ATR in response to replication stress) was dominated by MCM (Minichromosome Maintenance protein complex) and RFC (Replication Factor C). Intriguingly, human DNA replication factors MCM associate with EBV replication origin for the replication of its own genome thus potentially serving as a direct link to the viral pathogenesis of this disorder. Importantly, proteins in cluster 1, which dominantly represents TEN, and are only mildly elevated in MPR and DRESS, are associated almost exclusively with type I and II interferon signaling. Given the urgent clinical need for a specific therapy for TEN, it was focused on the role of the interferon pathway in TEN.

### 1.4 JAK/STAT pathway is drastically activated in TEN

Severe inflammatory disorders are often characterized by feedback loops that exacerbate the immune response and it was therefore hypothesized that proteins significantly regulated in both keratinocytes and immune cells would identify therapeutically relevant pathways. Six such proteins (WARS1, STAT1, S100A9, Lysozyme, GBP1 and APOL2) were identified that were all at least four-fold upregulated in both keratinocytes and immune cells of TEN compared to healthy (Fig 7a). Strikingly, all six proteins are part of the interferon pathway, responsible for signal transduction (STAT1) or triggered by it. Interferons signal through the Janus Kinase / Signal Transducer and Activator of Transcription (JAK/STAT) and it was set out to dissect its molecular components using our comprehensive cell-type resolved proteomic dataset (Fig 7b).

STAT1 and STAT3 were upregulated in both immune cells and keratinocytes, while STAT2 was up in keratinocytes and STATS in immune cells. Most downstream interferon-stimulated genes (ISG) were either strongly upregulated in comparison to healthy, illustrated by a 9-fold increase of GBP1, or even solely detectable in TEN. Substantial and broad JAK/STAT activation was confirmed in SJS/TEN by transcriptomic analysis in a second CADR cohort (N=27, Fig. 7c).

Of cytokines known to signal through the JAK/STAT pathway, interferon-gamma gene expression was the most strongly upregulated in TEN, exceeding that of cytokines such as TNF alpha known to be involved in TEN (Fig. 7d). Visualization of STAT1 in FFPE tissue sections further confirmed its significant upregulation in TEN (Fig. 7e). Notably, the degree of single-cell nuclear STAT1 upregulation in TEN was similar to DRESS, in which therapeutic intervention with JAK-inhibitor Tofacitinib was successful. To further establish STAT1 activation in TEN at the signaling level, it was proceeded with histological analysis of phosphorylated STAT1. This revealed a profound degree of STAT1 phosphorylation in both skin residing immune cells and keratinocytes of TEN patients (Fig. 7f).

Example 2 In-vitro model: Evaluation of JAK-inhibitor Tofacitinib as an inhibitor of immune-cell mediated cytotoxicity of keratinocytes in Steven-Johnson Syndrom / Toxic epidermal necrolysis

### 2.1 The JAK/STAT pathway is an actionable therapeutic target in TEN

To investigate the clinical significance of the experimental findings, a novel in-vitro model was developed that authentically replicates key aspects of cutaneous drug reactions (Fig 8a). To this end, we cultivated primary keratinocytes from single hair follicles of TEN survivors, building on previous protocols (Wustner, L. S. et al. Generating iPSCs with a High-Efficient, Non-Invasive Method-An Improved Way to Cultivate Keratinocytes from Plucked Hair for Reprogramming. Cells 11 (2022)). Concurrently, patient-matched PBMCs were isolated and activated using CD3/28. After five days, the keratinocytes were fluorescently labeled and co-cultured with activated or resting PBMCs. A fluorescent DNA-binding dye was introduced to identify cells with compromised membrane integrity, in order to visualize, track and quantify the cytotoxic action of activated PBMCs towards keratinocytes using live-cell imaging. In this autologous co-culture model, keratinocytes were efficiently killed within 72 hours in the presence of activated PBMCs (Fig. 8b).

Applying the pan-JAK inhibitor Tofacitinib resulted in dose-dependent inhibition of keratinocyte cytotoxicity, whereas co-culturing of keratinocytes with resting PBMCs or exposing of keratinocytes to Tofacitinib alone did not induce keratinocyte cell death (Fig 8b).

### 2.2 Method in detail:

Description of a novel co-culture, live-cell imaging assay using matched patient derived keratinocytes and immune cells for in detail molecular analysis of cutaneous drug reactions and cutaneous, immune-mediated processes in general.

### Generation of hair follicle- derived keratinocytes for co-culture assay

Hair follicles were placed on thin-Matrigel (Corning, #354234) coated T25 flasks and covered with 10 µl of 1.8 mg/ml Matrigel to prevent hair follicle detachment. Following one hour incubation at 37 °C / 5% CO2, 1ml of conditioned mouse embryonic fibroblast medium, from irradiated J2-3T3 cells, supplemented with 10 µM Y-27632 (CST #13624), 10 ng/ml FGF2 (Cell Guidance, #GFH146-50) was carefully added. Medium was changed every 48 hours. When outgrowth of keratinocytes became visible, medium was aspirated and replaced with 2ml Keratinocyte Expansion Medium DermaCult^{™} (Stemcell #100-0500, with supplement), and changed every 48 hours. When confluent, keratinocytes were detached using TrypLE^{™} Express Enzyme (Gibco #12604039) and defined trypsin inhibitor (Gibco # R007100) and replated on collagen-I (Corning, 354236) coated plates for further expansion.

### Isolation of patient PBMCs for co-culture assay

PBMCs were isolated using Ficol according to standard protocol. Cells were frozen in liquid nitrogen until further usage.

### Live-cell imaging co-culture assay

Patient PBMCs (200'000 / well / 200 µl) were stimulated in U-shape 96-well plates in the presence or absence of CD3/28 (Gibco Dynabeads, #11161D) at a ratio of 1:1, in X-VIVO 15 (Lonza, #881026) for 5 days at 37 °C / 5% CO2. On day 4 of PBMC stimulation, hair-follicle derived keratinocytes from the same patient were plated on Collagen-I coated 96-well plates at a density of 5'000 - 10000 / well (depending on the proliferation rate, patient specific). On day 5 of PBMC stimulation, keratinocytes were fluorescently labeled (Celltracker Red; Invitrogen #C34552) according to the manufacturer's recommendation. Activated or non-activated ("resting") PBMCs were then added to the keratinocytes at a ratio of 1:1, after magnetic removal of CD3/28 beads where necessary. Tofacitinib (CST, #14703) or control (DMSO) was added in the indicated concentration as well as Celltox Green (Promega, G8731, 1:8000 final dilution) to reach a final volume of 200 µl per well. Every 2 hours images were acquired using a live cell analysis system (Incucyte Sx3, Sartorius) for 72 hours at 37 °C / 5% CO2. Quantification of dead keratinocytes (Celltox Green+ / Celltracker Red+) was performed using the proprietary analysis software (Incucyte, Sartorius).

### Example 3 Mouse model

The efficacy of oral JAK inhibitors was evaluated in an established mouse model of TEN (Anderton, H., Rickard, J. A., Varigos, G. A., Lalaoui, N. & Silke, J. Inhibitor of Apoptosis Proteins (lAPs) Limit RIPK1-Mediated Skin Inflammation. J Invest Dermatol 137, 2371-2379 (2017).

### 3.1 Smac mimetic TEN mouse model:

As previously published (Anderton, H., Rickard, J. A., Varigos, G. A., Lalaoui, N. & Silke, J. Inhibitor of Apoptosis Proteins (lAPs) Limit RIPK1-Mediated Skin Inflammation. J Invest Dermatol 137, 2371-2379 (2017)) male mice were injected subcutaneously with 100µl of 1mg/ml smac mimetic (SM) CompA (TetraLogic Pharmaceuticals) or vehicle (12% Captisol). JAK inhibitor (10 mg/kg Baricitinib or 30 mg/kg Tofacitinib) or vehicle (20% Captisol; 50µl) was administered by oral gavage twice daily starting one day before s.c. SM injection. Mice were sacrificed on either day 1 or day 3 post SM injection, the site was photographed, scored, and samples collected for ex-vivo analysis. For mice where there was no lesion the approximate area of the injection site was collected.

### 3.3 Results

Firstly, loss of inhibitor of Apoptosis proteins (lAPs) shifts TNF signaling towards apoptosis and necroptosis and contributes to the pathology of TEN (Saito, N. et al. An annexin A1-FPR1 interaction contributes to necroptosis of keratinocytes in severe cutaneous adverse drug reactions. Sci Transl Med 6, 245ra295 (2014); Chung, W. H. & Hung, S. I. Recent advances in the genetics and immunology of Stevens-Johnson syndrome and toxic epidermal necrosis. J Dermatol Sci 66, 190-196 (2012)). In mice, subcutaneous (s.c.) injection of smac-mimetics (SM) which are known IAP antagonists, induced TEN-like cutaneous inflammation, epidermal detachment and histological features of TEN (Fig. 8c). Strikingly, a strong increase in pSTAT1 was detectable following SM injection, consistent with the human data in TEN (Fig. 8e). Oral treatment with JAKi (Tofacitinib 30 mg/kg or Baricitinib 10 mg/kg) markedly reduced macroscopic disease severity on day 1 and 3 as quantified by a clinical assessment score that includes all major hallmarks of TEN (Nikolsky sign, edema, rubor; Fig 8d). Reduced disease severity was accompanied by a significant reduction in cutaneous pSTAT1 levels and average dermal thickness as compared to vehicle (Fig 8f). JAKi also decreased keratinocyte cell death (TUNEL+/cleaved caspase 3+) and immune cell infiltration (Fig 8e).). Importantly, epidermal recovery following s.c. SM injection was accelerated in JAKi treated mice, demonstrated by the strong expression of the proliferation marker Ki67 in the basal layer, ruling out a detrimental effect of treatment on wound healing.

Taken together, this in vitro and in vivo data clearly demonstrate the efficacy of JAKi in preclinical models of TEN.

## Claims

1. A pharmaceutical composition comprising a Janus kinase (JAK) inhibitor for use for the treatment of toxic epidermal necrolysis (TEN) and/or Stevens-Johnson-syndrome (SJS) and/or SJS/TEN overlap.

2. The pharmaceutical composition of claim 1, wherein the Janus kinase (JAK) inhibitor is selected from tofacitinib, baricitinib, ruxolitinib, upadacitinib, abrocitinib, cerdulatinib, deucravacitinib, fedratinib, lestaurtinib, momelotinib, pacritinib, peficitinib, oclacitinib, delgocitinib, filgotinib, ritlecitinib, brepocitinib, decernotinib, itacitinib, gandotinib or gusacitinib.

3. The pharmaceutical composition according to claim 1 or 2, wherein the Janus kinase (JAK) inhibitor is tofacitinib or baricitinib.

4. The pharmaceutical composition according to any one of claims 1-3, wherein the Janus kinase (JAK) inhibitor is inhibiting JAK1, JAK2, JAK3 and/or TYK2.

5. The pharmaceutical composition according to any one of claims 1-4, wherein the pharmaceutical composition is administered orally or topically, in particular orally.

6. The pharmaceutical composition according to any one of claims 1-5, wherein the composition comprises the Janus kinase (JAK) inhibitor in an amount of from 1mg to 20 mg.

7. The pharmaceutical composition according to any one of claims 1-6, wherein the Janus kinase (JAK) inhibitor is administered in a daily amount of from 1 mg to 20 mg.

8. The pharmaceutical composition according to any one of claims 1-7, wherein the composition is administered twice daily, in particular for five days.

9. The pharmaceutical composition according to any one of claims 1-8, wherein the pharmaceutical composition further comprises at least one additional pharmaceutically active agent, which is in particular selected from ivlGs (intravenous immunoglobulins), TNFα-inhibitors, corticosteroids and/or with cyclosporin.

10. A Janus kinase (JAK) inhibitor for use for the treatment of toxic epidermal necrolysis (TEN) and/or Stevens-Johnson-syndrome (SJS) and/or SJS/TEN overlap.

11. A method for treatment of toxic epidermal necrolysis (TEN) and/or Stevens-Johnson-syndrome (SJS) and/or SJS/TEN overlap comprising application of a Janus kinase (JAK) inhibitor to a patient.
